# EUROPÄISCHE PATENTSCHRIFT

(11) **EP 2 725 093 B1**
(45) Veröffentlichungstag und Bekanntmachung des Hinweises auf die Patenterteilung: **07.08.2019**
(21) Anmeldenummer: 13190370.0
(22) Anmeldetag: 25.10.2013
(51) Int. Cl.: C12M 1/107, C12M 1/00, A01C 3/02, E04H 7/06

(54) **Biogasbehälter mit kuppelförmigem Tragluftdach**
Biogas container with a dome shaped air supported roof
Réservoir de bio gaz avec toiture gonflable en forme de coupole

(30) Priorität: 26.10.2012 DE 202012104130 U
(43) Veröffentlichungstag der Anmeldung: 30.04.2014
(73) Patentinhaber: JOPE Beteiligungs GmbH, 87653 Eggenthal (DE)
(72) Erfinder: Baur, Peter, 87724 Ottobeuren (DE); Baur, Josef, 87784 Westerheim-Rummeltshausen (DE)
(74) Vertreter: Pfister & Pfister Patent- und Rechtsanwälte

(56) Entgegenhaltungen:
- EP-A1- 2 502 478
- DE-A1- 2 522 453
- US-A- 3 839 832
- US-A- 4 902 304
- US-B1- 6 474 022

## Beschreibung

Die Erfindung betrifft einen Biogasbehälter mit kuppelförmigem Tragluftdach nach dem Oberbegriff des unabhängigen Anspruches 1.

Biogasbehälter werden innerhalb von Biogasanlagen eingesetzt, die zur Erzeugung von Biogas durch Vergärung von Biomasse dienen. In landwirtschaftlichen Biogasanlagen werden meist tierische Exkremente (Gülle, Festmist) und Energiepflanzen als Substrat eingesetzt. Bei den meisten Biogasanlagen wird das entstandene Gas vor Ort in einem Blockheizkraftwerk zur Strom- und Wärmeerzeugung genutzt.

Herkömmliche Biogasbehälter bestehen aus einem zylindrischen Grundkörper, der durch ein Dach, insbesondere Foliendach, abgedeckt ist, wobei das Foliendach entweder als einfache Siloabdeckung oder Doppelmembran-Siloabdeckung oder aber als Tragluftdach ausgebildet ist.

Bei derartigen Tragluftdächern wird mit dem äußeren gasdichten und witterungsbeständigen Foliendach und einer darunter liegenden Folie ein Luftpolster aufgebaut. Mit Hilfe eines Gebläses erzeugt dieses Luftpolster einen konstanten Druck im Inneren des Biogasbehälters. So ergibt sich eine typisch gewölbte Form des Tragluftdaches mit einer Druckdifferenz von 3 bis 5 Millibar. Unterhalb des Tragluftdaches kann eine tragende Konstruktion vorhanden sein, welche das Tragluftdach aufnimmt, falls das Gebläse ausfallen sollte, oder die äußeren Witterungsbedingungen das Tragluftdach nach unten in den Biogasbehälter drücken.

EP 2 502 478 A1 offenbart einen Biogasbehälter, der oberhalb der Folie verschiedene Tragstrukturen aufweist. Bei steigendem Innendruck im Biogasbehälter liegt die Folie dann an diesen Tragstrukturen an.

Aus US 6,474,022 B1 ist ein Abdecksystem mit einer Plane bekannt, welches auch für Biogasbehälter geeignet ist. Dieses Abdecksystem umfasst mit der Plane verbundene Seile, die zur Beschwerung oder Sicherung der Plane verwendet werden können.

US 4,902,304 A beschreibt einen doppelwandigen Gasbehälter, bei dem die äußere Folie bei ausreichendem Innendruck von innen an einer Verstärkungsstruktur aus Seilen anliegt.

Hier setzt die Erfindung an, welche sich zur Aufgabe gesetzt hat, ein gattungsgemäßes kuppelförmiges Tragluftdach für einen Biogasbehälter nach dem Stand der Technik derart weiterzubilden, dass das Tragluftdach sehr einfach und leicht, mit dennoch sehr hoher Festigkeit ausgestaltet ist.

Zur Lösung dieser Aufgabe dient die Merkmalskombination des unabhängigen Anspruches 1.

Wesentliches Merkmal hierbei ist, dass eine Vielzahl von Stabilisierungselementen mit einer Grundfolie lastübertragend zusammenwirkt, insbesondere wiederholt lösbar oder fest, das heißt zum Beispiel formschlüssig, kraftschlüssig oder stoffschlüssig verbunden sind, so dass die Zugkräfte über die Stabilisierungselemente abgeleitet werden. Erfindungsgemäß sind dafür die Stabilisierungselemente wenigstens abschnittsweise in taschenförmigen Hüllschläuchen aufgenommen, welche mit der Grundfolie verbunden, insbesondere unlösbar verbunden sind.

Wesentlicher Vorteil hierbei ist, dass durch das Vorsehen von Stabilisierungselementen die Zugkräfte und Auflagerkräfte der Grundfolie im Wesentlichen durch die Stabilisierungselemente selbst aufgenommen werden und somit eine sehr stabile Konstruktion eines kuppelförmigen Tragluftdaches bereit gestellt werden kann.

Weiterer wesentlicher Vorteil hierbei ist, dass durch das Vorsehen von Stabilisierungselementen die Eigenschaften (Material und/oder Dicke und Flächenmaße) der Grundfolie auf variable Zugkräfte und damit auf variable Betriebsdrucke des durch das Gebläse erzeugten Luftpolsters, sowie auf variable Auflagerkräfte auf den Behälter eingestellt werden kann.

Ein noch weiterer wesentlicher Vorteil besteht darin, dass die taschenförmigen Hüllschläuche einen zusätzlichen Sonnenschutz sowohl für die darin aufgenommenen Stabilisierungselemente als auch für die damit überdeckte Grundfolie darstellen. Gleichzeitig kann sich die Grundfolie zwanglos unterschiedlichen Befüllungsständen des Biogasbehälters anpassen, ohne dass durch die Stabilisierungselemente eine stets definierte Knickung der Grundfolie vorgegeben wäre, mit der Gefahr eines Bruches aufgrund einer nur punktuell oder linienförmig auftretenden Wechselbelastung. Eine derartige Wechselbelastung der Grundfolie wird insbesondere auch dadurch ausgeschlossen, dass die taschenförmigen Hüllschläuche nur abschnittsweise an der Grundfolie angebracht sind und/oder die Stabilisierungselemente nur abschnittsweise aufnehmen.

Ganz grundsätzlich bewirkt die Aufnahme der Stabilisierungselemente in den taschenförmigen Hüllschläuche darüber hinaus eine hohe Montagesicherheit und Betriebssicherheit, da eine Fehlverlegung oder ein Verrutschen der Stabilisierungselemente sowohl bei Montage als auch im nachfolgenden Betrieb eines erfindungsgemäßen Tragluftdachs ausgeschlossen sind. Durch die taschenförmigen Hüllschläuche wird zudem die Grundfolie vor potentiellen Beschädigungen durch die Stabilisierungselemente geschützt, dies insbesondere dann, wenn die Hüllschläuche unlösbar mit der Grundfolie verbunden sind.

Vorteilhafte Weiterbildungen sind Gegenstand der weiteren Unteransprüche.

Die Materialien der Grundfolie, sowie auch der Stabilisierungselemente sind vorzugsweise Kunststoff (auch als Textilien), könnten durchaus aber auch aus natürlichen Materialien wie zum Beispiel Holz oder Bambus gefertigt sein. Bevorzugt wird jedoch ein Kunststoff und insbesondere für die Stabilisierungselemente ein glasfaserverstärkter Kunststoff oder aber kohlefaserverstärkter Kunststoff, Kohlefaserverstärktem Kunststoff (KFK), Polyethylen (PE), Polypropylen (PP), Polyvinylchlorid (PVC), Polystyrol (PS), Polyurethan (PU), Polyethylenterephtalat (PET), Polytertafluorethylen (PTFE) oder Polymethylmethacrylat (PMMA). Alternativ ist vorgesehen, dass die Stabilisierungselemente aus einem (leichten) Metallmaterial gefertigt sind, und anschließend mit einem Kunststoffmaterial zum Schutz der Folie ummantelt werden.

Um das Gesamtgewicht des gesamten Tragluftdaches relativ gering zu halten, werden die Stabilisierungselemente band- oder gurtförmig gewählt, so dass ihre Länge in Relation zur Breite und Dicke relativ groß ist.

Die Stabilisierungselemente können auch elastisch federnd ausgebildet sein.

Auch kann hierdurch der Stützluftdruck erhöht werden, so dass dem negativen Einfluss der äußeren Witterungsbedingungen, insbesondere bei starkem Wind, damit entgegen gewirkt werden kann, so dass die Gefahr einer Beschädigung des Tragluftdaches vermindert wird.

Damit das gesamte Tragluftdach gleichmäßig stabil ausgebildet ist, sind natürlich die Stabilisierungselemente in etwa gleichen Abständen beziehungsweise Winkelabständen auf der Grundfolie angeordnet.

Insbesondere ist der Polbereich des kuppelförmigen Tragluftdaches durch Zugkräfte stark beansprucht, so dass hier die Dichte (d.h. Anzahl von Stabilisierungselemente pro Fläche) der Stabilisierungselemente relativ hoch sein sollte, so dass bevorzugt vorgesehen ist, dass in dem Polbereich des kuppelförmigen Tragluftdaches sich eine Vielzahl von Stabilisierungselementen dicht aneinander vorbeilaufen oder überlagern und dort miteinander wiederholt lösbar oder aber fest verbunden sind.

So bilden die Stabilisierungselemente der vorliegenden Erfindung eine stern- oder strahlenförmige Stabilisierungskonstruktion, wobei die Stabilisierungselemente sich vom Randbereich der Grundfolie in den mittleren Polbereich erstrecken und von dort wiederum in den Randbereich der Grundfolie weitergeführt werden. Damit wird erreicht, dass die gesamte Grundfolie von Stabilisierungselementen gleichmäßig durchzogen wird, wodurch eine besonders vorteilhafte Stabilisierung des kuppelförmigen Tragluftdaches erfolgt, bei gleichzeitig sehr leichter Konstruktion und besonders stabiler und strapazierfähiger Funktion, da für die Grundfolie ein relativ leichtes und einfaches Kunststoffmaterial gewählt werden kann, für die Stabilisierungselemente ebenfalls.

Erfindungsgemäß sind die Stabilisierungselemente auf der Grundfolie aufgebracht.

Für die Anbringung der Hüllschläuche an der Grundfolie gibt es wiederum verschiedene Möglichkeiten. So kann in einer ersten Ausführungsform vorgesehen sein, dass die Hüllschläuche aus dem Material der Grundfolie selbst gebildet sind und somit die Hüllschläuche nur separierte Kammern des Materials der Grundfolie bilden, die zum Beispiel durch Verschweißung oder Verklebung oder Vulkanisierung oder Vernähung gebildet werden.

In einer anderen Ausführungsform der vorliegenden Erfindung können die Hüllschläuche als separate Elemente auf die Grundfolie aufgelegt werden und dort ebenfalls stoffschlüssig oder formschlüssig verbunden werden, insbesondere durch Verschweißung, Verklebung, Vulkanisierung oder durch Vernähen.

Bevorzugt weist die Grundfolie ein Flächengewicht von 0,4 kg/m² bis zu 1,1 kg/m² auf, insbesondere von 0,5 kg/m² bis 0,9 kg/m², bevorzugt von 0,6 kg/m² bis 0,8 kg/m², insbesondere bevorzugt von 0,7 kg/m² ± 10 %. Im Vergleich mit herkömmlichen Grundfolien mit einem Gewicht von ungefähr 1,5 kg/m2 ist diese Grundfolie damit besonders leicht und kostengünstig. Das hohe Gewicht dieser Folie nach dem Stand der Technik ist auf den hohen Gewebeanteil zurückzuführen, der für eine entsprechende mechanische Stabilität bzw. Zugfestigkeit notwendig ist. Die deutlich leichteren Grundfolien, die bei dem erifndungsgemäßen Vorschlag einsetzbar sind, erleichtern auch erheblich die Montage, da ein deutlich geringeres Gewicht zu hantieren ist. Durch die wenigstens abschnittsweise Anbringung der Stabilisierungselemente in den taschenförmigen Hüllschläuchen kann deren potentiell verringerte Zugfestigkeit aber mindestens wieder ausgeglichen werden. Die Grundfolie muss deshalb auch nicht die herkömmlicherweise erforderlichen Zugfestigkeitswerte von ungefähr 10 kN über einen Querschnitt der Grundfolie von 0,05 m Breite aufweisen, sondern kann bevorzugt auf eine Zugfestigkeit von 1 kN bis 6 kN über diesen Querschnitt hinweg, insbesondere von 2 kN bis 4 kN, bevorzugt von 3 kN ± 15 % ausgelegt werden. Insbesonderen wird ein Intervall für die Zugfestigkeit der Grundfolie vorgeschlagen, das durch eine untere und eine obere Intervallgrenze beschrieben ist. Dabei bezieht sich die Zugfestigkeit immer auf eine Folienbreite der Grundfolie von 5 cm, also 0,05 m. Als untere Intervallgrenze werden dabei 1; 1,5; 2; 2,2; 2,4; 2,5; 2,55; 2,6; 2,7; 2,8 kN pro 5 cm Breite vorgesehen. Als Obergrenze des bevorzugten Intervalls werden folgende Werte vorgesehen: 3,2; 3,4; 3,45; 3,5; 3,6; 3,8; 4,0; 4,2; 4,5; 5,0; 5,5; 6,0 kN pro 5 cm Breite der Folie. Im Rahmen der Erfindung sind alle möglichen Kombinationen von Unter- und Obergrenzen dieses Intervalls mit offenbart und dem Fachmann in seiner Anwendung auch klar.

Die vorteilhafte Ausgestaltung der Grundfolie wird aber nicht nur durch Ihre Zugfestigkeit beschrieben, sondern in gleicher Weise auch durch ihr Flächengewicht, das auch in einem bevorzugten Intervall beschrieben ist. Dieses Intervall ist ebenfalls durch eine Intervalluntergrenze und eine Intervallobergrenze beschrieben, die Intervalluntergrenze beträgt 0,4; 0,5; 0,6; 0,63; 0,65; 0,7 kg/m², die Obergrenze beträgt 0,75; 0,77; 0,8; 0,9; 1,0; 1,1; 1,2; 1,3 kg/m<2>. Im Rahmen dieser Anmeldung sind hiermit alle möglichen Kombinationen von Unter- und Obergrenzen des Intervalls mit offenbart und dem Fachmann in seiner Anwendung auch klar.

Der Vorteil des Vorschlags tritt insbesondere bei einer geschickten Auswahl der Grundfolie mit entsprechend reduziertem Flächengewicht bzw. Zugfestigkeit zu Tage. Da die Zugfestigkeit bzw. die mechanische Belastbarkeit des Tragluftdaches nicht primär von der Grundfolie bewirkt, sondern von den Stabilisierungselementen geleistet wird, reduziert sich die vorzuhaltende Typenvielzahl der Grundfolie erheblich und daher auch entsprechende Lagerkosten. Gleichzeitig kann mit dem vorgeschlagenen Konzept eine Vielzahl unterschiedlicher Tragluftdächer realisiert werden, das heißt, die Mengen ein und derselben Grundfolie steigen, was in der Regel einhergeht mit sinkenden Bezugskosten. Neben dem günstigeren Grundfolienmaterial, welches nicht mehr die aufwendige mechanische Gewebeausstattung aufweisen muss, werden durch den Vorschlag auch gleichzeitig Skalierungseffekte erreicht, die den zusätzlichen Aufwand für die Stabilisierungselemente deutlich überwiegen. Der Vorschlag erreicht also ein stabileres und flexibleres Produkt bei günstigeren Herstellungskosten.

Erfindungsgemäß besteht die Grundfolie aus einem Gewebe und einer, insbesondere beidseitig vorgesehenen Beschichtung, um eine entsprechend hohe Zugfestigkeit selbst bei geringem Gewicht zu gewährleisten, und zudem eine hohe Dichtigkeit und Witterungsbeständigkeit aufzuweisen. Die Grundfolie bzw. die Beschichtung besteht vorzugsweise zumindest teilweise aus Polyvinylchlorid (PVC), Polyethylen (PE) oder Polytetrafluorethylen (PTFE), wodurch eine hohe Dichtigkeit, Witterungsbeständigkeit einschließlich UV-Beständigkeit und auch eine Beständigkeit gegen verschiedene Medien sowie Abriebfestigkeit eines erfindungsgemäßen Tragluftdachs gewährleistet ist. Im Hinblick auf das Anschmutzverhalten, aber auch auf die Lebensdauer eines solchen Tragluftdachs sind dabei unterschiedliche Oberflächenschlusslackierungen der Grundfolie denkbar. Dafür kann zum Beispiel eine Acryllackierung oder eine Polyvinylidenfluorid (PVDF)-Lackierung vorgesehen werden.

In der Zeichnung ist die Erfindung insbesondere in einem Ausführungsbeispiel schematisch dargestellt. Es zeigen:
- Fig. 1a:: eine Draufsicht auf den erfindungsgemäßen Biogasbehälter mit kuppelförmigem Tragluftdach;
- Fig. 1b:: eine dreidimensionale perspektivische Darstellung des erfindungsgemäßen Biogasbehälters mit kuppelförmigem erfindungsgemäßem Tragluftdach;
- Fig. 2:: einen Querschnitt durch einen Ausschnitt im Bereich eines Stabilisierungselementes.

In den Figuren sind gleiche oder einander entsprechende Elemente jeweils mit den gleichen Bezugszeichen bezeichnet und werden daher, sofern nicht zweckmäßig, nicht erneut beschrieben.

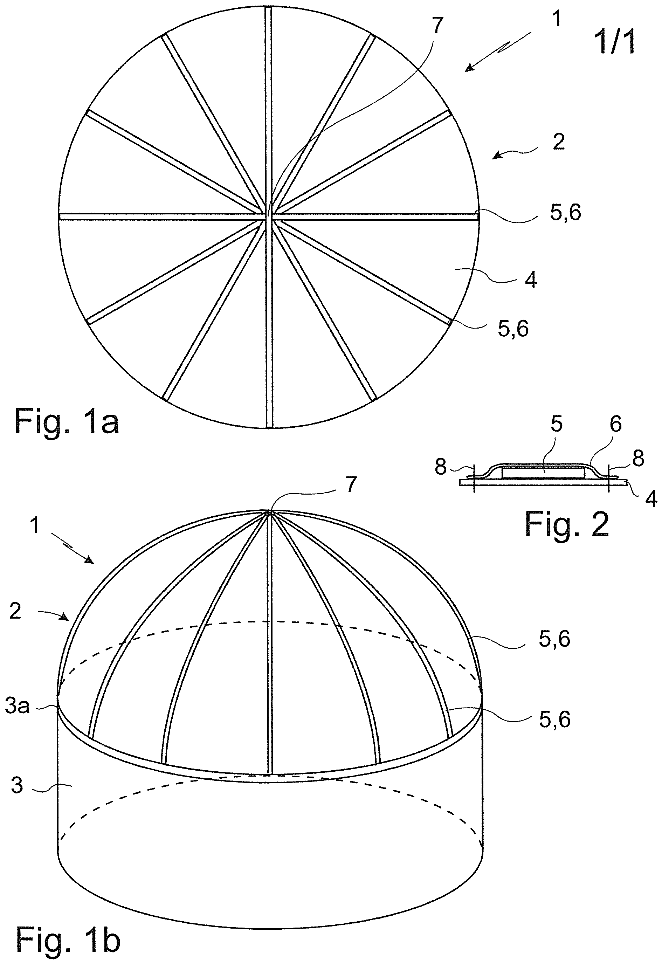

In Fig. 1b ist eine perspektivische Darstellung des erfindungsgemäßen kuppelförmigen Tragluftdaches für einen Biogasbehälter dargestellt.

Der erfindungsgemäße Biogasbehälter 1 beinhaltet das erfindungsgemäße Tragluftdach 2, welches auf der Oberseite 3a des Behältermantels 3 des Biogasbehälters aufgelegt ist. Hierbei stützen sich die Stabilisierungsgurte 5 des Tragluftdaches auf dieser Oberseite 3a des Behältermantels 3 ab und leiten somit die Zugkraft des gesamten Tragluftdaches ab auf den darunter befindlichen Behältermantel 3.

In Fig. la ist nun die Draufsicht auf den Biogasbehälter gemäß Fig. 1b zu sehen, wobei erkennbar ist, dass die variablen symmetrischen Stabilisierungsgurte 5 in Winkelabstand zueinander beabstandet auf der Grundfolie 4 aufgebracht sind, in zugeordneten Hüllschläuchen 6. Im Bereich des mittleren Poles 7 des Tragluftdaches 2 überdecken sich die jeweiligen Stabilisierungsgurte 5 und sind dort entweder lose aufeinander gelegt, oder aber miteinander wiederholt lösbar oder aber auch fest fixiert.

Die Fig. 2 zeigt nun einen Querschnitt im Bereich eines Stabilisierungsgurtes 5, welcher innerhalb des Hüllschlauches 6 aufgenommen ist, der sich auf der Grundfolie 4 befindet. Der Hüllschlauch 6 ist hierbei über Schweißpunkte 8 auf der Grundfolie 4 aufgebracht, wobei sich diese Schweißpunkte entlang der Längserstreckung der Stabilisierungsgurte 5 gleichmäßig erstrecken.

## Patentansprüche

1. Biogasbehälter mit kuppelförmigem Tragluftdach (2), wobei das Tragluftdach (2) eine gasdichte und gegebenenfalls auch flüssigkeitsdichte Grundfolie (4) beinhaltet, die auf der offenen Oberseite (3a) eines Behältermantels (3) mit insbesondere kreisrunder Grundform aufgebracht ist und eine Vielzahl von Stabilisierungselementen (5) vorgesehen ist, die lastübertragend mit der Grundfolie (4) zusammenwirken und einer Ableitung von Zugkräften dienen, wobei mehrere oder alle Stabilisierungselemente im Polbereich (7) der Grundfolie (4) zusammen eine stern- oder strahlenförmige Stabilisierungskonstruktion bilden, **dadurch gekennzeichnet, dass** die Stabilisierungselemente (S) auf der Grundfolie (4)aufgebracht sind und wenigstens abschnittsweise in taschenförmigen Hüllschläuchen (6) aufgenommen sind, welche mit der Grundfolie (4) verbunden, insbesondere unlösbar verbunden sind und die Grundfolie (4) aus einem Gewebe und einer Beschichtung besteht, wobei die Grundfolie (4) oder die Beschichtung zumindest teilweise aus Polyvinylchlorid (PVC), Polyethylen (PE) oder Polytetrafluorethylen (PTFE) besteht.

2. Biogasbehälter mit kuppelförmigem Tragluftdach (2) nach Anspruch 1, **dadurch gekennzeichnet, dass** die Grundfolie (4) aus Kunststoff besteht.

3. Biogasbehälter mit kuppelförmigem Tragluftdach (2) nach einem der vorhergehenden Ansprüche, **dadurch gekennzeichnet, dass** die Stabilisierungselemente (5) aus Kunststoff und/oder Metall bestehen, insbesondere aus Glasfaserverstärktem Kunststoff (GFK), Kohlefaserverstärktem Kunststoff (KFK), Polyethylen (PE), Polypropylen (PP), Polyvinylchlorid (PVC), Polyurethan (PU), Polytetrafluorethylen (PTFE) und/oder Aluminium oder Stahl.

4. Biogasbehälter mit kuppelförmigem Tragluftdach (2) nach einem der vorhergehenden Ansprüche, **dadurch gekennzeichnet, dass** die Stabilisierungselemente (5) band- oder gurtförmig ausgebildet sind, also relativ lang in Relation zur Breite und Dicke.

5. Biogasbehälter mit kuppelförmigem Tragluftdach (2) nach einem der vorhergehenden Ansprüche, **dadurch gekennzeichnet, dass** die Stabilisierungselemente (5) elastisch federnd ausgebildet sind.

6. Biogasbehälter mit kuppelförmigem Tragluftdach (2) nach einem der vorhergehenden Ansprüche, **dadurch gekennzeichnet, dass** die Stabilisierungselemente (5) gleichmäßig über die Fläche der Grundfolie (4) verteilt sind, insbesondere in etwa gleichen Abständen bzw. in etwa gleichen Winkelabständen und/oder die Stabilisierungselemente (5) sich vom Randbereich der Grundfolie (4) in den mittleren Polbereich (7) der Grundfolie (4) erstrecken.

7. Biogasbehälter mit kuppelförmigem Tragluftdach (2) nach einem der vorhergehenden Ansprüche, **dadurch gekennzeichnet, dass** die Stabilisierungselemente (5) jeweils lose oder formschlüssig, kraftschlüssig oder stoffschlüssig festgelegt sind.

8. Biogasbehälter mit kuppelförmigem Tragluftdach (2) nach einem der vorhergehenden Ansprüche, **dadurch gekennzeichnet, dass** die Hüllschläuche (6) aus dem Material der Grundfolie (4) gebildet sind oder mit der Grundfolie (4) stoffschlüssig oder formschlüssig verbunden sind, insbesondere verschweißt und/oder verklebt und/oder vulkanisiert und/oder vernäht sind.

9. Biogasbehälter mit kuppelförmigem Tragluftdach (2) nach einem der vorhergehenden Ansprüche, **dadurch gekennzeichnet, dass** die Verschweißung der Hüllschläuche (6) mit der Grundfolie (4) eine Punkt- oder Linien-Schweißung ist.

10. Biogasbehälter mit kuppelförmigem Tragluftdach (2)nach einem der vorhergehenden Ansprüche, **dadurch gekennzeichnet, dass** die Grundfolie (4) ein Flächengewicht von 0,4 kg pro Quadratmeter bis 1,1 kg pro Quadratmeter, insbesondere von 0,5 kg pro Quadratmeter bis 0,9 kg pro Quadratmeter, bevorzugt von 0,6 kg pro Quadratmeter bis 0,8 kg pro Quadratmeter, insbesondere bevorzugt von 0,7 kg pro Quadratmeter +/- 10% aufweist.

11. Biogasbehälter mit kuppelförmigem Tragluftdach (2) nach einem der vorhergehenden Ansprüche, **dadurch gekennzeichnet, dass** die Grundfolie (4) eine Zugfestigkeit von 1 kN bis 6 kN über einen Querschnitt der Grundfolie (4) von 0,05 m Breite, insbesondere von 2 kN bis 4 kN über einen Querschnitt der Grundfolie (4) von 0,05 m Breite, bevorzugt von 3 kN +/- 15% über einen Querschnitt der Grundfolie (4) von 0,05 m Breite aufweist.

## Claims

1. Biogas container with a dome shaped air supported roof (2), wherein the air supported roof (2) contains a gas-proof and, if necessary, also liquid-proof basic film (4) applied to the open surface (3a) of a container shell (3) with in particular circular basic shape, and a multitude of stabilizing elements (5) is provided that interact with the basic film (4) while transmitting load and serve for diverting tensile powers, wherein several or all stabilizing elements together in the pole area (7) of the basic film (4) form a star- or beam-like stabilizing construction, **characterized in that** the stabilizing elements (S) are attached to the basic film (4), and at least in sections are held in pocket-like enveloping hoses (6) that are connected to the basic film (4), in particular connected non-releasably, and the basic film (4) consists of a fabric and a coating, wherein the basic film (4) or the coating consists at least partly of polyvinyl chloride (PVC), polyethylene (PE), or polytetrafluorethylene (PTFE).

2. The biogas container with a dome shaped air supported roof (2) according to claim 1, **characterized in that** the basic film (4) consists of synthetic material.

3. The biogas container with a dome shaped air supported roof (2) according to any one of the preceding claims, **characterized in that** the stabilizing elements (5) consist of synthetic material and/or metal, in particular of glass fiber reinforced synthetic material (GFK), carbon fiber reinforced synthetic material (KFK), polyethylene (PE), polypropylene (PP), polyvinyl chloride (PVC), polyurethane (PU), polytetrafluorethylene (PTFE), and/or aluminum or steel.

4. The biogas container with a dome shaped air supported roof (2) according to any one of the preceding claims, **characterized in that** the stabilizing elements (5) are designed like tapes or belts, i.e. they are rather long in relation to their width or thickness.

5. The biogas container with a dome shaped air supported roof (2) according to any one of the preceding claims, **characterized in that** the stabilizing elements (5) are designed such that they spring elastically.

6. The biogas container with a dome shaped air supported roof (2) according to any one of the preceding claims, **characterized in that** the stabilizing elements (5) are distributed evenly across the surface of the basic film (4), in particular with identical spaces or roughly identical angular distances, and/or the stabilizing elements (5) extend from the edge zone of the basic film (4) to the center pole area (7) of the basic film (4).

7. The biogas container with a dome shaped air supported roof (2) according to any one of the preceding claims, **characterized in that** each of the stabilizing elements (5) is defined loosely or in a positive-locking, form-fitting or firmly bonded manner.

8. The biogas container with a dome shaped air supported roof (2) according to any one of the preceding claims, **characterized in that** the enveloping hoses (6) are formed of the material of the basic film (4), or are connected with the basic film (4) in a firmly bonded or positive locking manner, in particular welded and/or glued and/or cured-on and/or sewed.

9. The biogas container with a dome shaped air supported roof (2) according to any one of the preceding claims, **characterized in that** the welding of the enveloping hoses (6) with the basic film (4) is a spot or line welding.

10. The biogas container with a dome shaped air supported roof (2) according to any one of the preceding claims, **characterized in that** the basic film (4) has a weight per unit area of 0.4 kg per square meter to 1.1 kg per square meter, in particular 0.5 kg per square meter to 0.9 kg per square meter, preferably 0.6 kg per square meter to 0.8 kg per square meter, in particular preferred 0.7 kg per square meter +/- 10 %.

11. The biogas container with a dome shaped air supported roof (2) according to any one of the preceding claims, **characterized in that** the basic film (4) has a tensile strength of 1 kN to 6 kN across a cross section of the basic film (4) of 0.05 m of width, in particular 2 kN to 4 kN across a cross section of the basic film (4) of 0.05 m of width, preferably of 3 kN +/-15 % across a cross section of the basic film (4) of 0.05 m of width.

## Revendications

1. Réservoir de biogaz avec toiture gonflable en forme de coupole (2), réalisé de façon à ce que la toiture gonflable (2) comporte un film de base (4) étanche au gaz et, le cas échéant, étanche au liquide, placée sur la face ouverte supérieure (3a) d'une forme en particulier circulaire d'une paroi du réservoir (3) comportant de nombreux éléments stabilisateurs (5) interagissant par transmission de force avec le film de base (4) en servant à évacuer des forces de traction, et à ce que plusieurs ou tous les éléments stabilisateurs forment ensemble au niveau de la zone polaire (7) du film de base (4) une construction stabilisante en forme d'étoile ou de rayon, **caractérisé en ce que** les éléments stabilisateurs (S) sont situés sur le film de base (4) et sont au moins en partie logés dans des enveloppes tubulaires (6) en forme de poches liées au film de base (4) et en particulier liées de façon permanente et **en ce que** le film de base (4) est constitué en un tissu et en un revêtement de façon à ce que le film de base (4) ou le revêtement est constitué au moins en partie en chlorure de polyvinyle (PVC), en polyéthylène (PE), ou en polytétrafluoroéthylène (PTFE).

2. Réservoir de biogaz avec toiture gonflable en forme de coupole (2) selon la revendication 1, **caractérisé en ce que** le film de base (4) est en matière plastique.

3. Réservoir de biogaz avec toiture gonflable en forme de coupole (2) selon une des revendications précédentes, **caractérisé en ce que** les éléments stabilisateurs (5) sont en matière plastique et/ou en métal, en particulier en plastique renforcé par des fibres de verre (GFK), en plastique renforcé par des fibres de carbone (KFK), en polyéthylène (PE), en Polypropylène (PP), en chlorure de polyvinyle (PVC), en polyuréthane (PU), en polytétrafluoroéthylène (PTFE) et/ou en aluminium ou en acier.

4. Réservoir de biogaz avec toiture gonflable en forme de coupole (2) selon une des revendications précédentes, **caractérisé en ce que** les éléments stabilisateurs (5) possèdent des formes de bandes ou de courroies, c'est-à-dire qu'ils sont bien plus longs que larges.

5. Réservoir de biogaz avec toiture gonflable en forme de coupole (2) selon une des revendications précédentes, **caractérisé en ce que** les éléments stabilisateurs (5) possèdent les caractéristiques d'un ressort élastique.

6. Réservoir de biogaz avec toiture gonflable en forme de coupole (2) selon une des revendications précédentes, **caractérisé en ce que** les éléments stabilisateurs (5) sont distribués uniformément sur la face du film de base (4) et en particulier à des distances identiques ou à des distances d'angles identiques et/ou **en ce que** les éléments stabilisateurs (5) s'étendent de la zone de bord du film de base (4) vers la zone polaire (7) du milieu du film de base (4).

7. Réservoir de biogaz avec toiture gonflable en forme de coupole (2) selon une des revendications précédentes, **caractérisé en ce que** les éléments stabilisateurs (5) sont chacun immobilisés sans lien, par lien de forme, par lien de force, par lien par la matière.

8. Réservoir de biogaz avec toiture gonflable en forme de coupole (2) selon une des revendications précédentes, **caractérisé en ce que** les enveloppes tubulaires (6) sont fabriquées dans le matériau du film de base (4) ou à ce qu'elles sont liées au film de base (4) par lien de matière ou par lien de forme et en particulier soudées et/ou collées et/ou vulcanisées et/ou cousues.

9. Réservoir de biogaz avec toiture gonflable en forme de coupole (2) selon une des revendications précédentes, **caractérisé en ce que** la soudure des enveloppes tubulaires (6) au film de base (4) est réalisée par une soudure par points ou par lignes.

10. Réservoir de biogaz avec toiture gonflable en forme de coupole (2) selon une des revendications précédentes, **caractérisé en ce que** le film de base (4) possède un poids surfacique allant de 0,4kg par mètre carré à 1,1kg par mètre carré, en particulier allant de 0,5kg par mètre carré à 0,9kg par mètre carré, de préférence allant de 0,6kg par mètre carré à 0,8kg par mètre carré et en particulier et de préférence de 0,7kg par mètre carré +/-10%.

11. Réservoir de biogaz avec toiture gonflable en forme de coupole (2) selon une des revendications précédentes, **caractérisé en ce que** le film de base (4) possède une résistance à la traction allant de 1 kN à 6 kN appliquée à une section du film de base (4) d'une largeur de 0,05m, en particulier allant de 2 kN à 4 kN appliquée à une section du film de base (4) d'une largeur de 0,05m et de préférence de 3 kN +/-15% appliquée à une section du film de base (4) d'une largeur de 0,05m.
